**Europäisches Patentamt**

**European Patent Office**

**Office européen des brevets**

(19)

(11) Veröffentlichungsnummer : **0 405 097 B1**

(12) **EUROPÄISCHE PATENTSCHRIFT**

(45) Veröffentlichungstag der Patentschrift :
**02.12.92 Patentblatt 92/49**

(51) Int. Cl.$^5$ : **A61M 39/00, F16K 7/06**

(21) Anmeldenummer : **90108477.2**

(22) Anmeldetag : **05.05.90**

(54) **Gleitklemme für einen Schlauch.**

(30) Priorität : **30.06.89 DE 3921585**

(43) Veröffentlichungstag der Anmeldung :
**02.01.91 Patentblatt 91/01**

(45) Bekanntmachung des Hinweises auf die
Patenterteilung :
**02.12.92 Patentblatt 92/49**

(84) Benannte Vertragsstaaten :
**FR GB IT**

(56) Entgegenhaltungen :
**DE-C- 3 631 411**
**US-A- 2 715 402**
**US-A- 2 928 391**
**US-A- 3 900 184**
**US-A- 4 307 869**

(73) Patentinhaber : **NPBI Nederlands
Produktielaboratorium voor
Bloedtransfusieapparatuur en
Infusievloeistoffen B.V.
Runde ZZ41
NL-7881 HM Emmer Compascuum (NL)**

(72) Erfinder : **Biekart, Frank Theodoor
Hunenoord 34
NL-7822 BP Emmen (NL)**
Erfinder : **Hilbrink, Hubertus Eduard
Kuifmees 65
NL-7827 BP Emmen (NL)**

(74) Vertreter : **Masch, Karl Gerhard, Dr. Dipl.-Phys.
et al
Patentanwälte Andrejewski, Honke & Partner
Theaterplatz 3 Postfach 10 02 54
W-4300 Essen 1 (DE)**

**Beschreibung**

Die Erfindung betrifft eine Gleitklemme für einen Schlauch insbesondere eines Systems mit einem flexiblen Flüssigkeitsbehälter, bestehend aus einem Längskörper mit einer kreisförmigen Ausnehmung und einem daran über einen Überleitungsabschnitt anschließenden Längsschlitz für die Aufnahme des geöffneten bzw. abgeklemmten Schlauches siche z.B DE-C- 3 631 411.

Solche Gleitklemmen setzt man zum Abklemmen von Flüssigkeiten führenden flexiblen Schläuchen ein. Bevorzugtes Anwendungsgebiet sind Transfusions- und Infusionsanordnungen sowie Verbindungsschläuche von Blutbehältersystemen. Alle in der Praxis bekannten Gleitklemmen der eingangs genannten Art basieren auf demselben Prinzip. Sie bestehen aus einem gegebenenfalls am Rand verstärkten flachen Streifen als Längskörper, bei dem der Längsschlitz in der Ebene der kreisförmigen Ausnehmung liegt. Ein typischer Nachteil dieses Konzepts besteht darin, daß die Gleitklemme verhältnismäßig groß und nach Montage an einem Schlauch beispielsweise eines Blutbehältersystems aufgrund ihrer Sperrigkeit schwer zu verpacken ist. Insbesondere bei Vakuumverpackung verursachen die Gleitklemmen oftmals Zerstörungen der flexiblen Behälter.

Der Erfindung liegt daher die Aufgabe zugrunde, für eine Gleitklemme der eingangs genannten Art einen Aufbau zu finden, der zu verringerten Abmessungen führt und eine kompakte Verpackung zuläßt.

Die erfindungsgemäße Lösung dieser Aufgabe besteht darin, daß der Längskörper aus einer Winkelgestalt besteht, bei der die Ebene der kreisförmigen Ausnehmung senkrecht zum Längsschlitz angeordnet ist.

Der durch die Erfindung erreichte Vorteil besteht zunächst darin, daß die Gesamtlänge der Gleitklemme zufolge deren Winkelgestalt nur noch etwa 60 Prozent der Gesamtlänge der bekannten Gleitklemme ausmacht. In der Schlauchoffenstellung verläuft die Gleitklemme praktisch vollständig parallel zum Schlauch, wodurch sie ohne die Gefahr von Zerstörungen sehr dicht verpackt werden kann.

Für die weitere Ausgestaltung bestehen im Rahmen der Erfindung mehrere Möglichkeiten. So ist nach einer bevorzugten Ausführungsform die kreisförmige Ausnehmung von einem Längszylinderteil gebildet, welches als "Schlauchöffner" verwendbar ist, d. h. mit dem durch Verschieben ein flachgedrückter Schlauch wieder in seine ursprüngliche offene Rundgestalt überführt werden kann. Eine weitere bevorzugte Ausführungsform ist dadurch gekennzeichnet, daß der Längskörper im Bereich der kreisförmigen Ausnehmung mit einer Schlaucheinführungsöffnung versehen ist; das ist an sich nicht neu, im vorliegenden Fall aber ohne Inkaufnahme einer ins Gewicht fallenden Verringerung der Klemmstabilität realisierbar. Günstig ist es auch, die erfindungsgemäße Gleitklemme bzw. deren Längskörper als Spritzgußteil auszuführen, weil die Trennungslinie zwischen den beiden Spritzgußformteilen die bei den bekannten Gleitklemmen zu einem Schlauchbeschädigungen verursachenden scharfen Grat im Bereich des Längsschlitzes führt, bei der erfindungsgemäßen Gleitklemme außerhalb des Längsschlitzes angeordnet werden kann.

Im folgenden wird die Erfindung anhand einer Zeichnung näher erläutert; es zeigen

Fig. 1 in perspektivischer Darstellung eine erste Ausführungsform einer Gleitklemme,

Fig. 2 in perspektivischer Darstellung eine zweite Ausführungsform einer Gleitklemme,

Fig. 3 eine Aufsicht auf eine dritte Ausführungsform einer Gleitklemme

Fig. 4 eine Vorderansicht der Gleitklemme gemäß Fig. 3 in Richtung des Pfeiles A,

Fig. 5 einen Schnitt B-B durch die Gleitklemme gemäß Fig. 3, und

Fig. 6 schematisch die Funktionsweise einer Gleitklemme.

Die in den Figuren dargestellten Gleitklemmen sind zum Aufsetzen auf einen Schlauch bestimmt. Der Schlauch kann Bestandteil eines Systems mit einem flexiblen Flüssigkeitsbehälter, wie beispielsweise von Transfusions- oder Infusionssets oder Blutbehältersystemen, sein; der Schlauch könnte aber auch Bestandteil eines Systems mit starren Flüssigkeitsbehältern, z. B. aus Glas, sein. In ihrem grundsätzlichen Aufbau bestehen die Gleitklemmen jeweils aus einem Längskörper 1 mit einer kreisförmigen Ausnehmung 2 und einem daran über einen Überleitungsabschnitt 3 anschließenden Längsschlitz 4 für die Aufnahme des geöffneten bzw. abgeklemmten Schlauches. Bei allen Ausführungsformen ist die Anordnung so getroffen, daß die Ebene der kreisförmigen Ausnehmung 2 senkrecht zum Längsschlitz 4 angeordnet ist. Auf diese Weise ist die Gleitklemme bei durch die Ausnehmung 2 verlaufendem Schlauch praktisch an den Schlauch anlegbar. Zum Abklemmen des Schlauches wird die Gleitklemme um 90° verschwenkt und der Schlauch über den Überleitungsabschnitt 3 in den Längsschlitz 4 eingeführt und eingeklemmt.

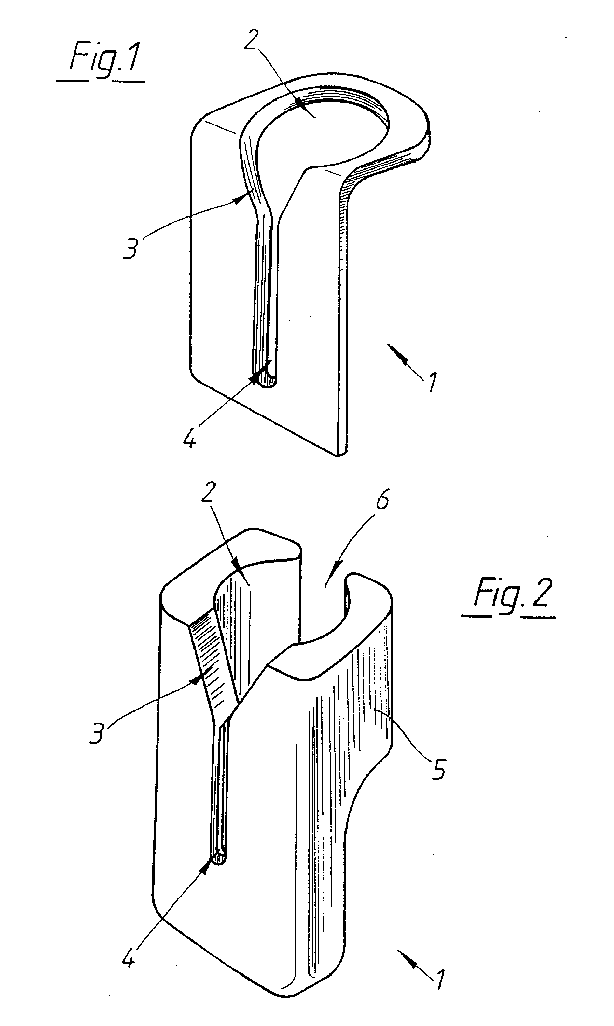

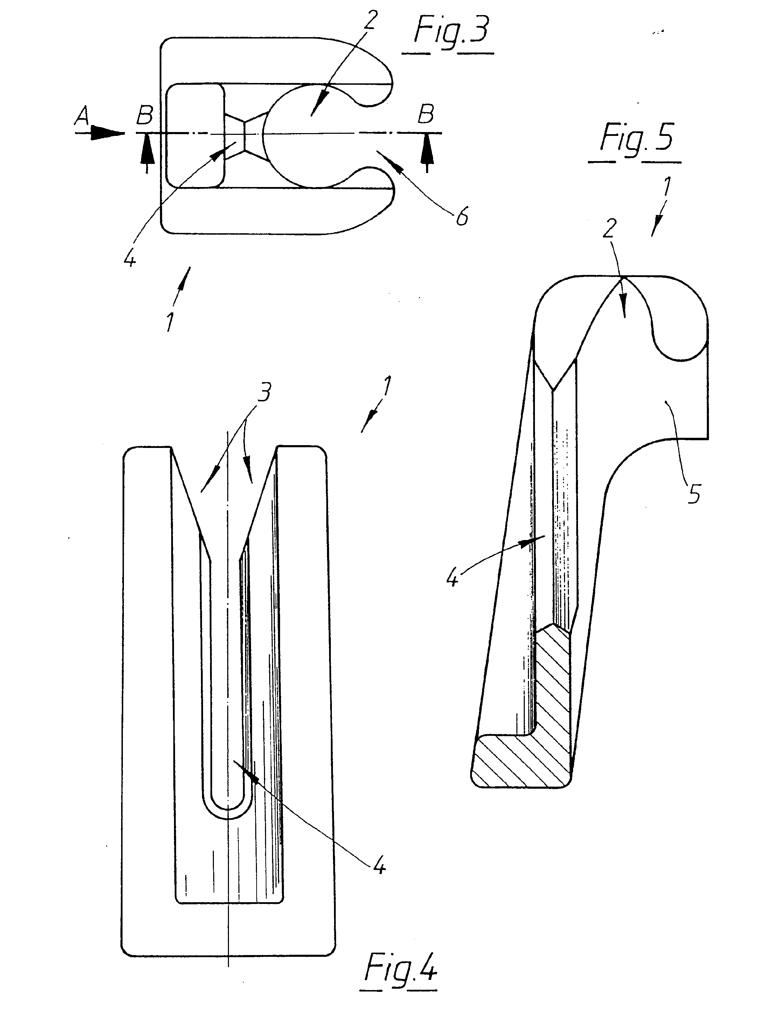

Bei der Ausführungsform nach Fig. 1, die besonders kostengünstig herstellbar ist, besteht der Längskörper 1 aus einer abgewinkelten streifenförmigen Platte aus thermoplastischem Kunststoff. Bei den Ausführungsformen nach den Fig. 2 bis 5 ist dagegen der Längskörper 1 jeweils als Spritzgußteil aus thermoplastischem Kunststoff ausgeführt. Die kreisförmige Ausnehmung 2 ist dabei von einem Längszylinderteil 5 gebildet, mit dem ein zusammengekniffen bleibender Schlauch durch Relativverschiebung geöffnet werden kann. Im übrigen erkennt man in den Fig. 2 bis 5, daß der Längskörper 1 im Bereich der kreisförmigen Ausnehmung 2 mit einer Schlaucheinführungsöffnung 6 versehen ist, und zwar auf der dem Längsschlitz 4 gegenüberliegenden Seite.

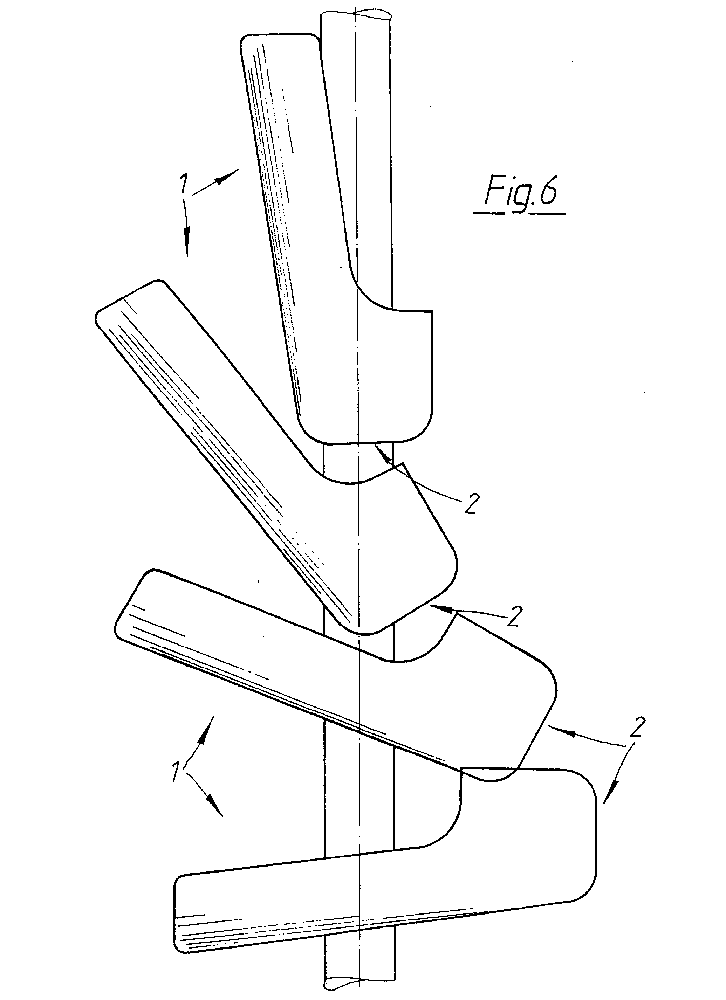

Aus der Fig. 6 entnimmt man ohne weiteres, wie die Gleitklemme aus der Offenstellung in die Schließstellung überführt wird.

## Patentansprüche

1. Gleitklemme für einen Schlauch insbesondere eines Systems mit einem flexiblen Flüssigkeitsbehälter, bestehend aus einem Längskörper (1) mit einer kreisförmigen Ausnehmung (2) für die Aufnahme des geöffneten Schlauches und einem an die Ausnehmung (2) über einen Überleitungsabschnitt (3) anschließenden Längsschlitz (4) für die Aufnahme des abgeklemmten Schlauches, **gekennzeichnet durch** eine Winkelgestalt des Längskörpers (1), bei der die Ebene der kreisförmigen Ausnehmung (2) senkrecht zum Längsschlitz (4) angeordnet ist.

2. Gleitklemme nach Anspruch 1, dadurch gekennzeichnet, daß die kreisförmige Ausnehmung (2) von einem Längszylinderteil (5) gebildet ist.

3. Gleitklemme nach Anspruch 1 oder 2, dadurch gekennzeichnet, daß der Längskörper (1) im Bereich der kreisförmigen Ausnehmung (2) mit einer Schlaucheinführungsöffnung (6) versehen ist.

4. Gleitklemme nach einem der Ansprüche 1 bis 3, dadurch gekennzeichnet, daß der Längskörper (1) als Spritzgußteil mit außerhalb des Längsschlitzes (4) verlaufender Formtrennungslinie ausgeführt ist.

## Claims

1. A sliding clamp for a flexible tube, particularly of a system with a flexible liquid container, comprising a longitudinal body (1) with a circular recess (2) to receive the opened flexible tube and a longitudinal slot (4), attached to the recess (2) via a transition section (3), to receive the clamped-off flexible tube, characterised by an angular shape of the longitudinal body (1) in which the plane of the circular recess (2) is disposed perpendicularly to the longitudinal slot (4).

2. A sliding clamp according to Claim 1, characterised in that the circular recess (2) is formed from a longitudinal cylinder part (5).

3. A sliding clamp according to Claims 1 or 2, characterised in that the longitudinal body (1) is provided with an aperture (6) for the introduction of a flexible tube, in the region of the circular recess (2).

4. A sliding clamp according to any one of Claims 1 to 3, characterised in that the longitudinal body (1) is constructed as an injection-moulded part with the mould separation line running outside the longitudinal slot (4).

## Revendications

1. Pince-tube à coulisse pour un tuyau flexible, en particulier d'un système avec un réservoir à liquides flexible, comprenant un corps longitudinal (1) avec un évidement circulaire (2) pour la réception du tuyau flexible ouvert suivi, par l'intermédiaire d'une section de transition (3), d'une fente longitudinale (4) pour la réception du tuyau flexible pincé, **caractérisé par le fait** que le corps longitudinal (1) présente une forme coudée pour laquelle le plan de l'évidement circulaire (2) est disposé perpendiculairement à la fente longitudinale (4).

2. Pince-tube à coulisse selon la revendication 1, caractérisé par le fait que l'évidement circulaire (2) est constitué par un élément cylindrique longitudinal (5).

3. Pince-tube à coulisse selon l'une des revendications 1 ou 2, caractérisé par le fait que le corps longitudinal (1) présente dans la région de l'évidement circulaire (2) une ouverture (6) pour l'introduction du tuyau flexible.

4. Pince-tube à coulisse selon l'une des revendications 1 à 3, caractérisé par le fait que le corps longitudinal (1) est réalisé sous la forme d'une pièce moulée par injection avec une ligne de séparation du moule située à l'extérieur de la fente longitudinale (4).

_Fig.1_

_Fig.2_

Fig.3

Fig.5

Fig.4

_Fig.6_

1

1

2

2

2